# EUROPEAN PATENT SPECIFICATION

(11) **EP 0 553 590 B1**
(45) Date of publication and mention of the grant of the patent: **04.06.1997**
(21) Application number: 92500036.6
(22) Date of filing: 07.04.1992
(51) Int. Cl.: C07D 311/46, A61K 31/35

(54) **Antithrombotics derived from coumarin and process for obtaining them.**
Antithrombotika, die sich von Cumarin ableiten, und Verfahren zu ihrer Herstellung
Antithrombotiques dérivés de coumarine et procédé pour leur préparation

(30) Priority: 31.01.1992 ES 9200196
(43) Date of publication of application: 04.08.1993
(73) Proprietor: LABORATORIOS FARMACEUTICOS ROVI, S.A., 28037 Madrid (ES)
(72) Inventor: Lopez Belmonte, Lorenzo, ES-28470 Cercedilla (Madrid) (ES)
(74) Representative: Hernandez Covarrubias, Arturo

(56) References cited:
- GB-A- 1 175 808
- GB-A- 2 055 831
- US-A- 2 427 578
- CHEMICAL ABSTRACTS, vol. 83, no. 25, 22 December 1975, Columbus, Ohio, US; abstract no. 206102g, page 378 ;
- CHEMICAL ABSTRACTS, vol. 98, no. 13, 28 March 1983, Columbus, Ohio, US; abstract no. 102730d, page 222 ;

## Description

This invention relates to new derivatives of hydroxy-4-coumarin, of the general formula 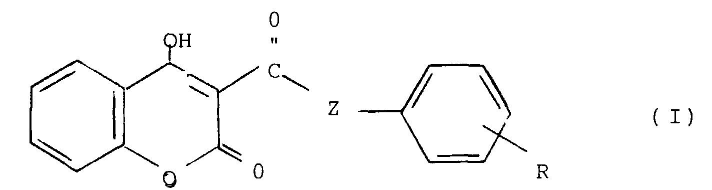 wherein Z can be -(CH₂)ₙ-O-, 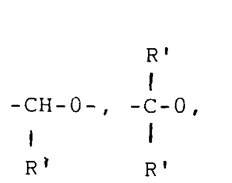 wherein n is 1 or 2 and R' is H, CH₃ or C₂H₅, and R can be H, CH₃, C₂H₅, Cl, Br, F, NO₂, CF₃.

The position of R is preferably para or meta.

These derivatives are resultant of the acylation in the 3-position of the hydroxy-4-coumarin.

JP-A-50 046 666 describes coumarin derivatives of the formula: 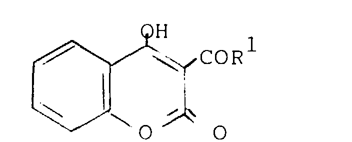 wherein R¹ is -(CH=CH)ₙ- R or -(CH₂)₂ₙR wherein R is an aromatic group and n is 1-2. Said coumarins have antibacterial, hypotensive and antiinflammatory activities.

CS-B- 200796 describes coumarin derivatives of the formula: 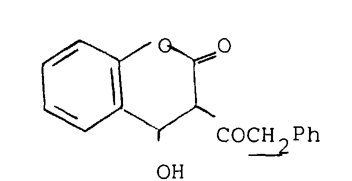 which have rodenticide activity.

US-A-2 427 578 describes anticoagulant coumarin derivatives of the formula: 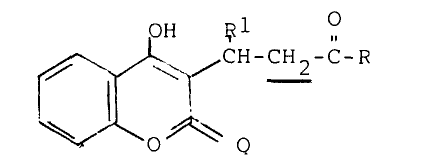 wherein R can be (i.a.) a phenyl group.

GB-A- 1 175 808 describes anticoagulant coumarin derivatives of the formula: 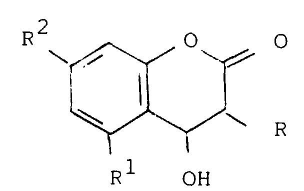 wherein R is a phenyl or alpha-naphthyl radical, or a phenyl or alpha-naphthyl radical substituted with at least one alcoxy group having from 1 to 5 carbon atoms.

GB-A- 2 055 831 describes rodenticide coumarin derivatives of the formula: 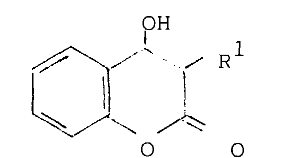 wherein R¹ is -CO-Y or

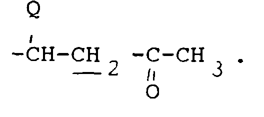

The general preparation procedure of this invention consists in effecting the acylation with an acid chloride according to the following scheme:

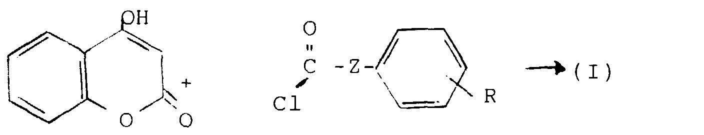

The reaction is preferably effected in pyridine, in the presence of piperidine.

The products according to the invention possess a potent anticoagulant and antithrombotic activity that has been evidenced in both the rat and in the rabbit.

These products can be used as antithrombotic agents for the prevention and treatment of tromboembolic and coronary diseases.

The invention will be further described by the following examples with reference to the attached drawings in which:

Figure 1 shows the IR spectrum of the compound obtained according to the example 1.

Figure 2 shows the NMR spectrum of the compound obtained according to the example 2.

Figure 3 is a graph that shows the anticoagulant activity in the rat of the compounds according to this invention, in ordinates being showed the degree of prothrombin and in abscises being showed the ingestion time in hours.

Figure 4 is a graph that shows the anticoagulant activity in the rabbit of the compounds according to this invention, in ordinates being showed the degree of prothrombin and in abscises being showed the ingestion time in hours.

The following examples illustrate the invention without limiting its extent.

### EXAMPLE 1

3-(4-methyl phenoxy isopropionyl)-4-hydroxy coumarin

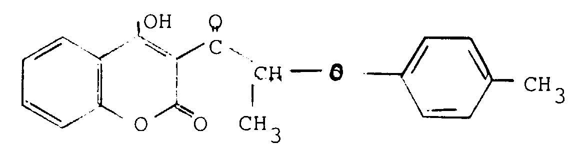

### Preparation

In 110 ml of anhydrous pyridine, introduce 12 drops of piperidine, 13,5 gr of 4-hydroxy coumarin and 23,3 g of 2-(4-methyl phenoxy)-propionyl chloride. Heat during 3 hours at 95^{º} 5^{º}C. Once same returns to room temperature, slowly pour the reactional mixture into 700 ml of HCl 2N and 350 g of ice. A brown precipitate becomes generated which is isolated by filtration. The product is crystallized in absolute ethanol (330 ml) in presence of active coal. Accordingly, 10.6 g of a beige product is obtained.

### Analysis

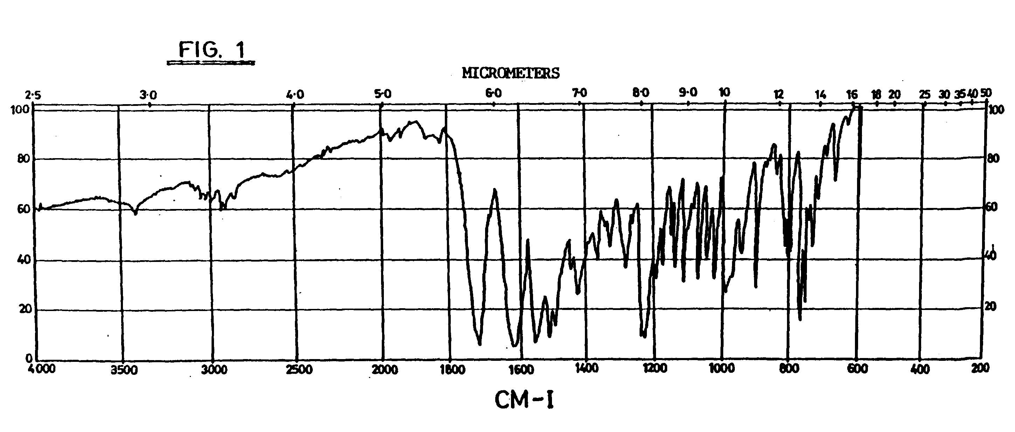

Melting point: 155-156^{º}C
Analysis:
   IR Spectrum at 200 MHz: see figure 1

### EXAMPLE 2

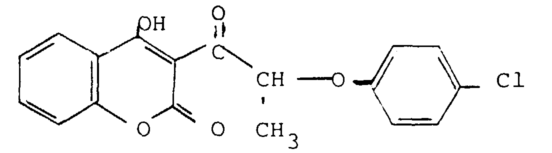

### Preparation

The method of preparation is identical to that of example 1. By causing the 2-(4-chloro phenoxy)-propionyl chloride to react with hydroxy-4-coumarin, a beige product with a yield of 51% is obtained after crystallization in butanol.

### Analysis

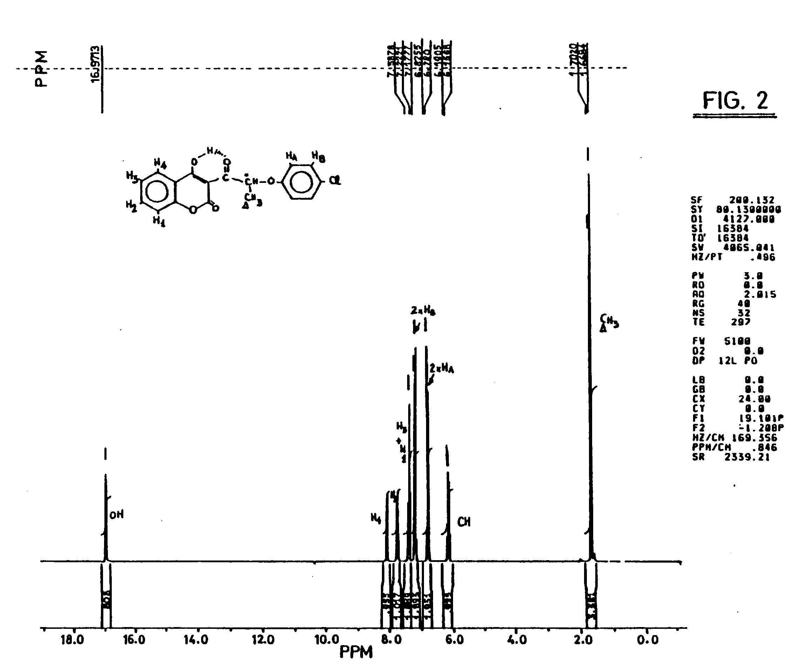

Melting point: 179.180^{º}C
Titratable chloride: 10.42% (theory:10.3)
NMR Spectrum at 200 MHz: see the figure 2

### Anticoagulant Activity

The measurements were made in the rat and the rabbit working on a homogeneous bath of 6 animals.

The product has been administrated by mixing same with corn starch, in suspension in an Arabic gum mucilage.

Blood from the anesthetized animal is taken and the Quick time is then determined in the plasm, according to the customary technique. A previous calibration, effected in a control plasma, enables one to evaluate the degree of prothrombin, and for a given dose, the curve indicating the decrease in the degree of prothrombin can be plotted againts a time interval.

This hypothrombinat action characterizes the antivitamin K activity of the products according to the invention.

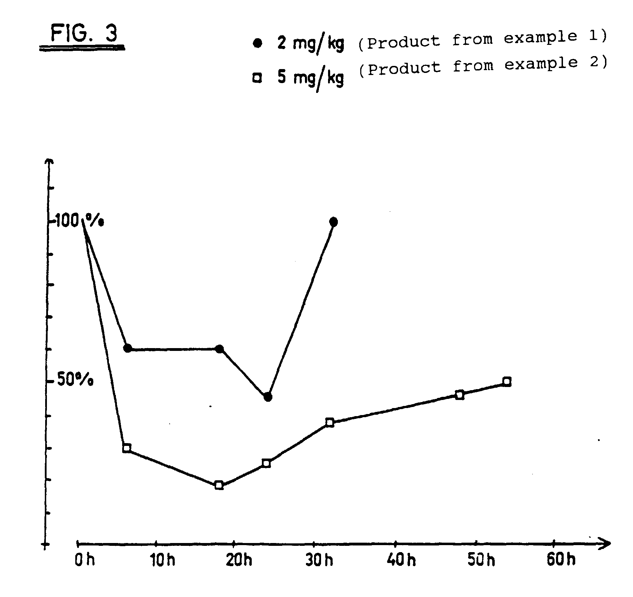

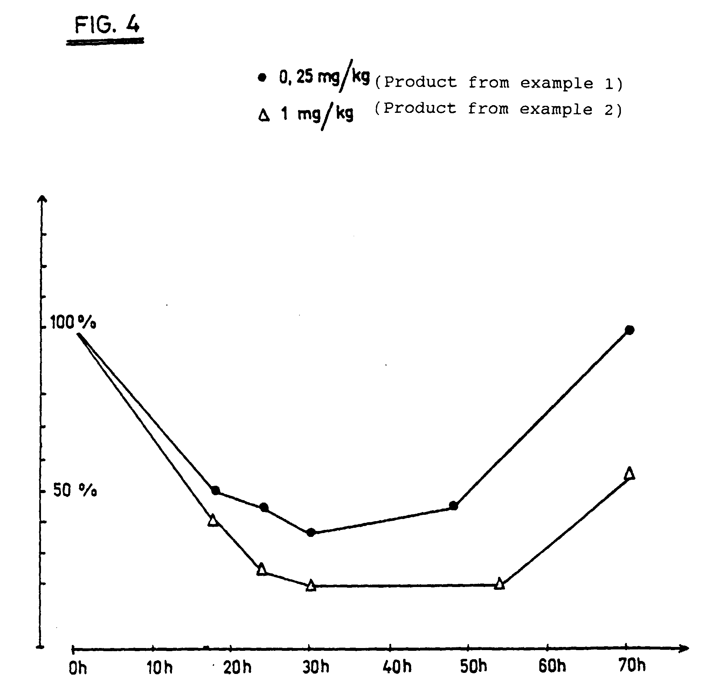

The curves obtained show the great activity of these products for an active dose of 2 mg/kg in the rat and of 0.2 mg/kg in the rabbit (figures 3 and 4).

## Claims

1. Acylated derivatives of the hydroxy-4-coumarin of the general formula (I) 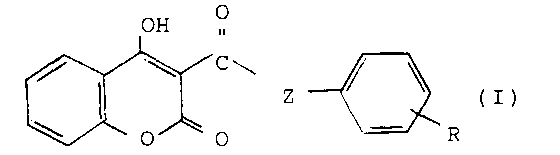 wherein z can be =(CH₂)ₙ-O-, 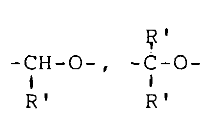 wherein n is 1 or 2 and R' is H, CH₃ or C₂H₅, and R can be H, CH₃, C₂H₅, Cl, Br, F, NO₂, CF₃, endowed with anticoagulant and antithrombotic properties.

2. 3-(4-methyl phenoxy isopropionyl)-4-hydroxy coumarin.

3. 3-(4-chloro phenoxy isopropionyl)-4-hydroxy coumarin.

4. Procedure for the preparation of the new acylated derivatives of hydroxy-4-coumarin of the general formula (I) indicated in the claim 1, characterized in that it comprises acylating hydroxy-4-coumarin with an acid chloride of the formula 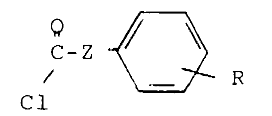 wherein Z and R are defined as mentioned in claim 1, preferably in pyridine, and in the presence of piperidine.

5. Medicines bases on the active principle of the general formula (I), useable as antithrombotic agents for the prevention and treatment of arterial and venous thrombosis.

6. Medicines that contain as their active principle 3-(4-methyl phenoxy isopropionyl)-4-hydroxy coumarin.

7. Medicines that contain as their active principle 3-(4-chloro phenoxy isopropionyl)-4-hydroxy-coumarin.

## Patentansprüche

1. Acylierte Hydroxy-4-Cumarin Derivate der allgemeinen Formel (I) 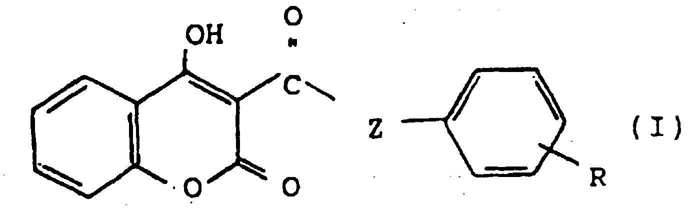 worin Z
=(CH₂)ₙ-O-, 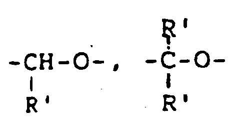
sein kann,
n 1 oder 2 ist,
R' = H, CH₃ oder C₂H₅ ist
und R H, CH₃, C₂H₅, Cl, Br, F, NO₂, CF₃ jeweils ausgestattet mit anti- koagulierenden und anti-thrombotischen Eigenschaften sein kann.

2. 3-(4-methyl phenoxy isopropionyl)-4-Hydroxy-Cumarin.

3. 3-(4-chlor phenoxy isopropionyl)-4-Hydroxy-Cumarin.

4. Verfahren zur Zubereitung neuer acylisierter Hydroxy-4-Cumarin Derivate der allgemeinen Formel (I) gemäß Anspruch 1, dadurch gekennzeichnet, daß es Acyl-Hydroxy-4-Cumarin mit einer Chloridsäure der Formel 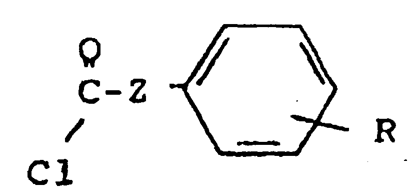 umfaßt,worin Z und R gemäß Anspruch 1, vorzugsweise in Pyridin und in Anwesenheit von Piperidin definiert sind.

5. Heilmittel basierend auf dem aktiven Prinzip der allgemeinen Formel (I), verwendbar als antithrombotische Mittel zur Verhütung und Behandlung von Arterien- und Venenthombose.

6. Heilmittel, die als aktives Prinzip 3-(4-methyl phenoxy isopropionyl)-4-Hydroxy-Cumarin enthalten.

7. Heilmittel, die als aktives Prinzip 3-(4-chlor phenoxy isopropionyl)-4-Hydroxy-Cumarin enthalten.

## Revendications

1. Dérivés acylés d'hydroxy-4-coumarine de la formule générale 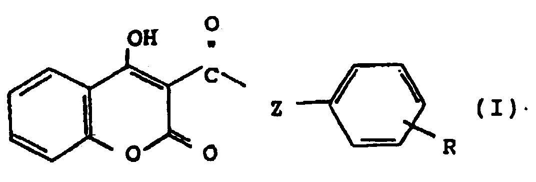 dans laquelle z peut être -(CH₂)ₙ-O-, 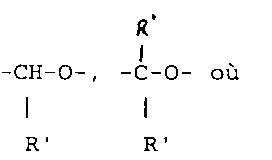 n est 1 ou 2 et R' est H, CH₃ ou C₂H₅, et R peut être H, CH₂, C₂H₅, Cl, Br, F, NO₂, CF₃, dotés de propriétés anticoagulantes et antithrombotiques.

2. 3-(4-méthyl phénoxy isopropionyl)-4-hydroxycoumarine.

3. 3-(4-chloro phénoxy isopropionyl)-4-hydroxycoumarine.

4. Procédé pour la préparation des nouveaux dérivés acylés d'hydroxy-4-coumarine de formule générale (I) indiquée dans la revendication 1, caractérisé en ce qu'il comprend l'acylation d'hydroxy-4-coumarine avec un chlorure d'acide de formule 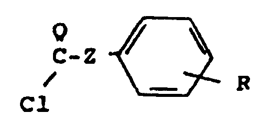 dans laquelle Z et R sont définis comme indiqué dans la revendication 1, de préférence dans de la pyridine et en présence de pipéridine.

5. Médicaments basés sur le principe actif de formule générale (I), pouvant être utilisés comme agents antithrombotiques pour la prévention et le traitement de thromboses artérielles et veineuses.

6. Médicaments qui contiennent comme principe actif de la 3-(4- méthyl phénoxy isopropionyl)-4-hydroxycoumarine.

7. Médicaments qui contiennent comme principe actif de la 3-(4- chloro phénoxy isopropionyl)-4-hydroxycoumarine.
